# EUROPÄISCHE PATENTSCHRIFT

(11) **EP 2 463 359 B1**
(45) Veröffentlichungstag und Bekanntmachung des Hinweises auf die Patenterteilung: **28.08.2013**
(21) Anmeldenummer: 10015464.0
(22) Anmeldetag: 09.12.2010
(51) Int. Cl.: C12M 1/107

(54) **Großraumbehälter**
Large container
Récipient à grande capacité

(43) Veröffentlichungstag der Anmeldung: 13.06.2012
(73) Patentinhaber: Drössler GmbH Umwelttechnik, 57080 Siegen (DE)
(72) Erfinder: Feldmann, Harald, 57489 Drolshagen (DE); Rossow, Norbert, Fritz, Bernhard, 17217 Penzlin (DE)
(74) Vertreter: Grosse, Wolf-Dietrich Rüdiger

(56) Entgegenhaltungen:
- EP-A1- 1 717 305
- EP-A1- 1 992 683
- EP-A1- 2 216 467
- DE-A1- 3 335 141
- DE-A1- 10 056 445
- DE-A1-102009 034 127
- GB-A- 1 218 245

## Beschreibung

### Großraumbehälter

Die Erfindung betrifft einen Großraumbehälter aus, auf einem Boden aufstehenden, miteinander verbundenen, aus vorgefertigten Betonteilen bestehenden Wandelementen, mit mindestens einer Zugangs-, Bedien- und Wartungsöffnung im folgenden Zugangsschacht und mit ggf. vorgesehener Behälterabdeckung.

Derartige Großraumbehälter werden in der Wasser- und Abwassertechnik sowie für Biogasanlagen und sonstigen landwirtschaftlichen Anlagen eingesetzt. Sie bestehen regelmäßig aus hochwertigem Beton, der entsprechend gas- und wasserdicht ausgebildet ist.

Die DE 197 14 342 C2 offenbart eine Biogasanlage zur Fermentation von organischen Stoffen, die einen Dom aufweist, der auf einer festen Fermenterdeckenwand gasdicht aufgeschraubt ist. Ein Einsatz ohne Decke bzw. bei Zelt- oder Tragluftdächern ist hier nicht möglich.

Die DE 10 2009 034 127 A1 offenbart eine Biogasanlage-Serviceeinrichtung, die von außen an eine Großraumbehälterwand angeschraubt ist und deren Serviceschacht über den Großraumbehälter ragt. Hier ist zwar eine Kombination mit einem Zeltdach- bzw. Tragluftdach etc. möglich, die in der Regel aus Edelstahl bestehende Serviceeinrichtung baut jedoch sehr groß und muss vor Ort aufwendig zusammengebaut werden.

Ähnliches gilt für den Biogasanlagen-Serviceschacht nach der EP 1 717 305 A1, welcher einen Serviceschacht offenbart, der mit der Innenwand über eine Tragkonsole verbunden ist. Auch hier ist ein aus vielen Einzelteilen bestehender Serviceschacht offenbart, der ebenso wie seine Tragkonsole aufwendig vor Ort zusammengebaut werden muss.

Der Erfindung liegt die Aufgabe zugrunde, einen Großraumbehälter mit einem Zugangsschacht so auszugestalten, dass der Zugangsschacht aus wenigen, vorgefertigten Teilen besteht, und unkompliziert und schnell vor Ort aufgebaut werden kann.

Zur Lösung dieser Aufgabe wird vorgeschlagen, dass mindestens ein Wandelement des Großraumbehälters gegenüber den übrigen Wandelementen höher ausgeführt ist, und dass zumindest über die Länge der Wandelementverlängerung zumindest ein im Wesentlichen 90° in den Behälterinnenraum ragender, bzw. auf die Behältermittelachse weisender Anschluss vorgesehen ist, dass der jeweilige Anschluss einstückig mit dem verlängerten Wandelement ausgebildet ist, und dass zwei der Anschlüsse über einen im Wesentlichen parallel zu den verlängerten Wandelementen angeordneten Abschluss zur Bildung eines Zugangsschachts verbunden sind.

Die einstückig mit den verlängerten Wandelementen verbundenen Anschlüsse geben dem Zugangsschacht eine hohe statische Festigkeit und erlauben hohe Belastungen, z. B. durch Abdeckung; Ausrüstung und Bedienpersonal. Da die Anschlüsse einstückig mit der Behälterwand ausgeführt sind, entfallen zusätzliche Abdichtungen zwischen Behälterwand und Schachtbauwerk. Die Konstruktion kann werksseitig weitestgehend vorgefertigt werden und wird dann gleichzeitig mit dem Behälter montiert. Hinzu kommt, dass der Behälterzugang sehr sicher ist, da lediglich über die Krone der verlängerten Behälterwand gestiegen werden muss, um an den Zugangsschacht zu gelangen. Zugangswege zum Schacht, die über den Behälter geführt sind, entfallen.

Ein besonderer Vorzug ergibt sich, wenn die zwei Anschlüsse und der Abschluss einstückig mit einem verlängerten Wandelemente ausgeführt sind.

Damit kann der komplette Zugangsschacht bereits vorgefertigt werden und ist mit der Aufstellung des verlängerten Wandelementes vor Ort sofort verfügbar.

Es besteht aber auch die Möglichkeit, dass je ein Anschluss an einem verlängerten Wandelement angeordnet ist, dass zumindest zwei der verlängerten Wandelemente nebeneinander angeordnet sind und die beiden Anschlüsse durch ein Abschlusselement verbunden sind.

Dadurch lassen sich beliebige Größen des Zugangsschachts verwirklichen.

Dabei ist vorteilhaft, dass das Abschlusselement von den Anschlüssen getragen und abgedichtet mit diesen verbunden ist.

Damit lässt sich eine leichte Montage verwirklichen. Es werden lediglich die beiden verlängerten Wandelemente mit den Anschlüssen nebeneinander aufgestellt. Der Abschluss kann nunmehr auf die Tragelemente der Anschüsse aufgesetzt werden und anschließend mit den Anschlüssen gedichtet verbunden werden.

Von Vorteil ist, dass der Zugangsschacht durch mindestens eine abnehmbare Abdeckung verschlossen ist. Die demontierbare Schachtabdeckung dient einerseits als Bedienungsplattform, kann aber für einen Zugang, für Beschickungszwecke oder z. B. für Räumgeräte wie Minibagger abgehoben werden. Die Abdeckung kann dabei außen bündig oder mit Überstand auf die Krone des Zugangsschachtes aufgesetzt oder aber in Rücksprünge in der Krone des Zugangsschachtes eingesetzt sein.

Vorzugsweise ist die Abdeckung aus einer eine Öffnung aufweisenden abnehmbaren Randabdeckung und einem, die Öffnung verschließenden, ebenfalls abnehmbaren Deckel gebildet. Bei Abnahme des Deckels ist der Zugangsschacht z. B. für Personen begehbar. Sollen größere Geräte in den Zugangsschacht eingebracht werden, kann die Randabdeckung zusätzlich abgenommen werden.

Dabei hat es sich als vorteilhaft erwiesen, dass zumindest die Randabdeckung ebenfalls aus einem Betonfertigteil besteht. Das Betonfertigteil kann bereits durch sein Eigengewicht leicht gasdicht auf den Zugangsschacht aufgebracht werden.

Ist eine Behälterabdeckung vorgesehen, kann diese vorzugsweise mit den in den Behälterinnenraum weisenden Wänden des Zugangsschachts gasdicht verbunden werden.

Die Erfindung wird anhand einer Zeichnung näher erläutert. Dabei zeigt:
- Figur 1: eine perspektivische Ansicht eines teilweise geschnitten dargestellten Ausschnitts eines Großraumbehälters mit einstückigem Zugangsschacht, und
- Figur 2: einen Zugangsschacht der sich über zwei verlängerte Wandelemente erstreckt.

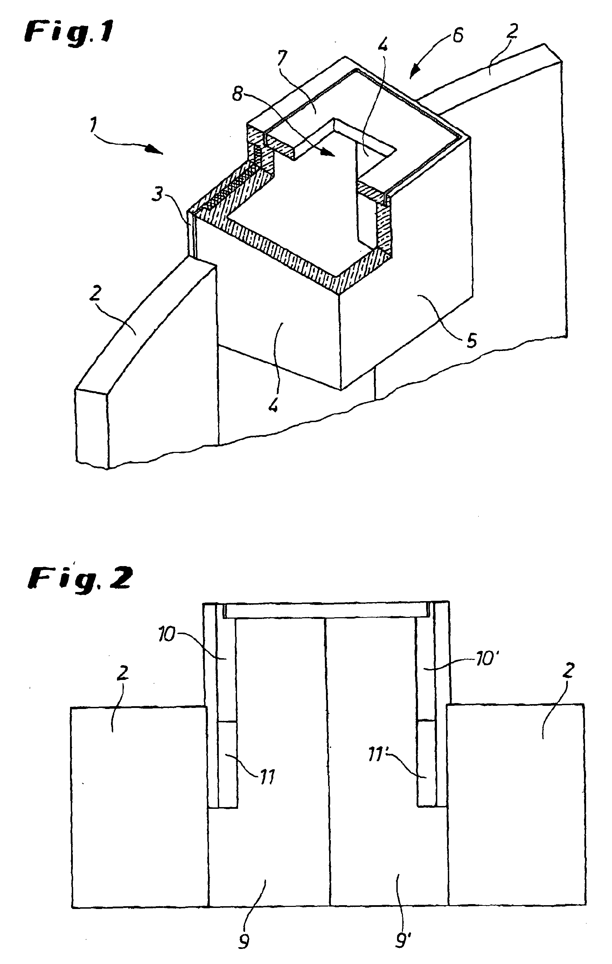

Figur 1 zeigt einen Ausschnitt eines Großraumbehälters 1, wobei Wandelemente 2, die auf einem nicht dargestellten Boden aufstehen zu erkennen sind. Ein verlängertes Wandelement 3 weist eine größere Höhe auf als die übrigen Wandelemente 2. Mit dem verlängerten Wandelement 3 sind Anschlüsse 4 einstückig verbunden. Ein Abschluss 5 ist mit den Anschlüssen 4 ebenfalls einstückig verbunden, so dass an dem verlängerten Wandelement 3 ein Zugangsschacht 6 einstückig angeformt ist.

In Rücksprünge der Krone des verlängerten Wandelements 3 sowie den Anschlüssen 4 und dem Abschluss 5 ist eine Randabdeckung 7 abnehmbar eingesetzt zu erkennen, die eine Öffnung 8 aufweist, die durch einen nicht dargestellten Deckel verschließbar ist.

Der Zugangsschacht 6, bestehend aus dem verlängerten Wandelement 3 sowie den Anschlüssen 4 und dem Abschluss 5 ist werksseitig als ein Betonfertigelement ausgebildet, und damit sofort nach Montage des verlängerten Wandelements 3 verfügbar. Dabei kann die Höhe der Anschlüsse 4 an dem verlängerten Wandelement 3 und die Höhe des Abschlusses 5 je nach Bedarf gewählt werden.

Es besteht aber auch die Möglichkeit, wie Figur 2 zeigt, zwei verlängerte Wandelemente 9, 9' vorzusehen, die jeweils einen einstückig mit ihnen verbundenen Anschluss 10, 10' aufweisen. Die Anschlüsse 10, 10' weisen Tragelemente 11, 11' auf, auf die ein ebenfalls entsprechend ausgeklinktes, nicht dargestelltes Abschlusselement aufsetzbar ist.

### Bezugszeichenübersicht

- 1: Großraumbehälter
- 2: Wandelemente
- 3: verlängerte Wandelemente
- 4: Anschluss
- 5: Abschluss
- 6: Zugangsschacht
- 7: Randabdeckung
- 8: Öffnung
- 9: verlängertes Wandelement
- 10: Anschluss
- 11: Tragelement

## Patentansprüche

1. Großraumbehälter (1) aus, auf einem Boden aufstehenden, miteinander verbundenen, aus vorgefertigten Betonteilen bestehenden Wandelementen (2, 3; 9), mit mindestens einer Zugangs-, Bedien- und Wartungsöffnung, im Folgenden Zugangsschacht (6), und mit ggf. vorgesehener Behälterabdeckung,
**dadurch gekennzeichnet,**
**dass** mindestens ein Wandelement (3, 9) des Großraumbehälters gegenüber den übrigen Wandelementen (2) höher ausgeführt ist, und dass zumindest über die Länge der Wandelementverlängerung zumindest ein im Winkel von 90° Grad in den Behälterinnenraum ragender Anschluss (4; 10) vorgesehen ist, dass der jeweilige Anschluss (4; 10) einstückig mit dem verlängerten Wandelement (3; 9) ausgebildet ist, und dass zwei der Anschlüsse (4; 10) über einen im Wesentlichen parallel zu den verlängerten Wandelementen (3; 9) angeordneten Abschluss (5) zur Bildung eines Zugangsschachtes (6) verbunden sind.

2. Großraumbehälter nach Anspruch 1,
**dadurch gekennzeichnet,**
**dass** zwei Anschlüsse (4) und der Abschluss (5) einstückig mit dem verlängerten Wandelement (3) ausgebildet sind.

3. Großraumbehälter nach Anspruch 1,
**dadurch gekennzeichnet,**
**dass** je ein Anschluss (10, 10') an einem verlängerten Wandelement (9, 9') angeordnet ist, dass zwei der verlängerten Wandelemente (9, 9') nebeneinander angeordnet sind und dass die beiden Anschlüsse (10, 10') durch ein Abschlusselement verbunden sind.

4. Großraumbehälter nach Anspruch 3,
**dadurch gekennzeichnet,**
**dass** das Abschlusselement von den Anschlüssen (10, 10') getragen und abgedichtet und mit diesen verbunden ist.

5. Großraumbehälter nach einem der Ansprüche 1 bis 4,
**dadurch gekennzeichnet,**
**dass** der Zugangsschacht (6) durch mindestens eine abnehmbare Abdeckung verschlossen ist.

6. Großraumbehälter nach Anspruch 5,
**dadurch gekennzeichnet,**
**dass** die Abdeckung aus einer, eine Öffnung (8) aufweisenden abnehmbaren Randabdeckung (7) und einem, die Öffnung (8) verschließenden, ebenfalls abnehmbaren Deckel besteht.

7. Großraumbehälter nach Anspruch 6,
**dadurch gekennzeichnet,**
**dass** zumindest die Randabdeckung (7) aus einem Betonfertigteil besteht.

8. Großraumbehälter nach einem der Ansprüche 1 bis 7,
**dadurch gekennzeichnet,**
**dass** eine Behälterabdeckung mit den in den Behälterinnenraum weisenden Wänden des Zugangsschachts (6) gasdicht verbunden ist.

## Claims

1. A large-scale container (1) manufactured from wall elements (2, 3; 9) consisting of prefabricated interconnected concrete components standing upright on the ground with at least one access, operating and maintenance opening, called access shaft (6) in the following, and provided, if required, with a container covering, **characterised in that** at least one wall element (3, 9) of the large-scale container is constructed higher relative to the other wall elements (2) and that at least over the length of the wall element elongation, at least one connection (4; 10) is provided protruding into the container interior at an angle of 90°, **in that** the respective connection (4; 10) is formed in one piece with the elongated wall element (3; 9) and **in that** two of the connections (4; 10) are connected via a termination (5) arranged essentially parallel to the elongated wall elements (3; 9) for forming an access shaft (6).

2. The large-scale container according to claim 1 **characterised in that** two connections (4) and the terminating element (5) are formed in one piece with the elongated wall element (3).

3. The large-scale container according to claim 1 **characterised in that** respectively one connection (10, 10') is arranged on one elongated wall element (9, 9'), **in that** two of the elongated wall elements (9, 9') are arranged adjacent to each other and that the two connections (10, 10') are connected by a terminating element.

4. The large-scale container according to claim 3 **characterised in that** the terminating element is supported by the connections (10, 10'), and sealed and connected with the same.

5. The large-scale container according to one of claims 1 to 4, **characterised in that** the access shaft (6) is closed by at least one removable cover.

6. The large-scale container according to claim 5 **characterised in that** the cover consists of a removable edge cover (7) comprising an opening (8) and an also removable lid closing the opening (8).

7. The large-scale container according to claim 6 **characterised in that** at least the edge cover (7) consists of a prefabricated concrete component.

8. The large-scale container according to one of claims 1 to 7, **characterised in that** a container cover is connected in a gas-tight manner with the walls of the access shaft (6) pointing into the interior of the container.

## Revendications

1. Conteneur pour grands volumes (1) constitué d'éléments de paroi (2, 3 ;9) posés debout sur un fond, reliés les uns aux autres, en pièces préfabriquées en béton avec au moins une ouverture d'accès, de manipulation et de maintenance, appelée ci-dessous trappe de visite (6) et avec un recouvrement de conteneur prévu le cas échéant, **caractérisé en ce qu'**au moins un élément de paroi (3, 9) du conteneur pour grands volumes est conçu en étant plus haut que les autres éléments de paroi (2) et **en ce qu'**au moins sur la longueur du prolongement de l'élément de paroi, il est prévu au moins un raccord (4 ; 10) saillant sous un angle de 90°c dans l'espace intérieur du conteneur, **en ce que** le raccord (4 ; 10) concerné est conçu en monobloc avec l'élément de paroi (3 ; 9) prolongé et **en ce que** deux des raccords (4 ; 10) sont reliés par l'intermédiaire d'une terminaison (5) disposée sensiblement à la parallèle des éléments de paroi (3 ; 9) prolongés, pour la création d'une trappe de visite (6).

2. Conteneur pour grands volumes selon la revendication 1, **caractérisé en ce que** deux raccords (4) et la terminaison (5) sont conçus en monobloc avec l'élément de paroi (3) prolongé.

3. Conteneur pour grands volumes selon la revendication 1, **caractérisé en ce que** chaque fois un raccord (10, 10') est disposé sur un élément de paroi (9, 9') prolongé, **en ce que** des éléments de paroi (9, 9') prolongés sont disposés côte à côte et **en ce que** les deux raccords (10, 10') sont reliés par un élément de terminaison.

4. Conteneur pour grands volumes selon la revendication 3, **caractérisé en ce que** les éléments de terminaison sont portés par les raccords (10, 10') et sont reliés de manière étanche avec ces derniers.

5. Conteneur pour grands volumes selon l'une quelconque des revendications 1 à 4, **caractérisé en ce que** la trappe de visite (6) est fermée par au moins un recouvrement amovible.

6. Conteneur pour grands volumes selon la revendication 5, **caractérisé en ce que** le recouvrement est constitué d'un recouvrement de bord (7) amovible comportant une ouverture (8) et d'un couvercle également amovible, recouvrant l'ouverture (8).

7. Conteneur pour grands volumes selon la revendication 6, **caractérisé en ce qu'**au moins le recouvrement de bord (7) est constitué d'une pièce préfabriquée en béton.

8. Conteneur pour grands volumes selon l'une quelconque des revendications 1 à 7, **caractérisé en ce qu'**un recouvrement de conteneur est relié de manière étanche au gaz avec les parois de la trappe de visite (6) dirigées vers l'espace intérieur du conteneur.
